# EUROPEAN PATENT APPLICATION

(11) **EP 1 460 120 A1**
(43) Date of publication of application: **22.09.2004**
(21) Application number: 02788725.6
(22) Date of filing: 04.12.2002
(51) Int. Cl.: C09K 3/00, C09D 7/12, C09D 127/12, C08L 27/12, A61K 7/00, A61K 7/48, A61K 33/00, A61P 17/00

(54) **ANION GENERATING MATERIAL AND SKIN TREATING AGENT USING THE SAME**

(30) Priority: 27.12.2001 JP 2001396358; 28.08.2002 JP 2002249133
(71) Applicant: Litec Corporation, Kyoto-shi, Kyoto 604-0874 (JP)
(72) Inventor: HATANO, Teruei, Kyoto-shi, Kyoto 601-8206 (JP); KIMURA, Tsutomu, Kyoto-shi, Kyoto 616-8017 (JP); UCHIYAMA, Masakatsu, Kyoto-shi, Kyoto 615-8084 (JP)
(74) Representative: Morf, Jan Stefan, Dr. Dipl.-Chem.
(86) International application number: PCT/JP2002/012733
(87) International publication number: WO 2003/060034

(57) **Abstract**

A substance safe and efficiently generating negative ions can be provided by allowing a negative ion generator to contain a) powder of an ore having spontaneous polarization and b) an organofluororesin organic compound in a contact state where b) component is negatively charged. Preferably, b) component contains fluororesin organic compound powder negatively charged by electrical charging; a) component and b) component are both powder having a particle size of 1 mm or less; and the mixing ratio of a) component and b) component is 1/99 to 99/1. Here, a mixture of a) component and b) component can be used extremely effectively as a skin treatment material for eliminating skin pain.

## Description

### FIELD OF THE INVENTION

The present invention relates to a negative ion generator using powder of an ore having spontaneous polarization and to a skin treatment material using the same.

### BACKGROUND ART

"An atom or an atomic group such as a molecule having electric charge" is referred to as ion. Plenty of ions are present in nature and in ambient atmosphere. Ions having a positive electric charge are referred to as positive ions. Environments or air of positive ions are said to be present in a large amount in newly built houses, rooms having a lot of electric appliances, places having a lot of automobile exhaust gas, and smoking areas. It is said that these positive ions affect human beings to invite insufficiency of autonomic nerves, causing dizziness, headache, irritation, and nervous disease.

On the other hand, environments and air having plenty of negative ions are said to be present in a large amount around forests, woods, grass, waterfalls, and fountains in humid places, and in a spa. These places are said to be effective in the improvement of bodies and respiratory organs, mental stability, reduction in the feelings of fatigue, and immunity.

Therefore, environments having plenty of negative ions are preferred. As a method or substance for artificially generating and discharging negative ions, there is a method of corona discharge using electricity. Also, there is ozone-generating equipment, water breaking method by waterfalls,fountains,andsteam discharge, a method using an ore such as tourmaline, a method of radiation using a radioactive element such as bolonium or radon, and other methods.

These methods each have advantages and disadvantages; however, as a method or object that is highly safe, is easy to carry and lay, and easily generates and discharges negative ions, a method using an ore such as tourmaline is often in use.

As a method using a natural ore, one can mention tourmaline, zeolite, Roche salt, calcium strontium propionate, and others.

In particular, tourmaline is a substance having permanent electric polarization as an electric stone. A substance having permanent electric polarization generates negative ions in a moving state. However, single tourmaline is a substance that generates very weak negative ions amounting to almost nothing. In order to generate negative ions in a large amount, there are Davi ore, Carnot stone, Toll stone, Thorium stone, and the like constituting a powder that generates a slight amount of radiation. Japanese Laid-open Patent Publication No. 2001-20177 discloses a method of using a mixture of a powder of an ore partly containing these with a natural ore.

Furthermore, Japanese Laid-open Patent Publication No. 2001-288679 discloses a method of using a mixture with porous mud, diatomaceous earth, zeolite, or the like which is an inorganic porous material. However, all of these are inorganic substances, and discharge radiation though only slightly. Also, damage to human beings by inorganic metals is immeasurable.

Further, even if these are used as a mixture with tourmaline, the amount of negative ion generation increases only a little from a very weak state. Negative ion discharge of such an amount is easily neutralized by positive ions in a living environment or air, and it is hard to expect an effect that can give a favorable influence on living environment or human beings.

Nevertheless, products claiming to generate negative ions by allowing several percents of such tourmaline or the aforementioned substances in a superfine particle form to adhere to or be kneaded into resin or fiber are marketed in a large amount. However, these negative ions are further extremely weak, and are far from placing living environment or commodities always under negative ions.

Therefore, an object of the present invention is to eliminate the drawbacks of the prior art and to provide a substance that effectively generates negative ions attracting people' s attention as giving favorable influence on human beings substantially without using an ore that discharges radiation, though using an ore having spontaneous polarization, as well as to provide a skin treatment material that eliminates skin pain or the like by using the substance.

### DISCLOSURE OF THE INVENTION

The inventors of the present invention observed that the principle of negative ion generator of an ore having spontaneous polarization such as tourmaline is said to derive from its crystal structure, that it is said to be polarized into a site of a pointed positive pole at the tip of tourmaline crystal and a negative pole at a flat part of the crystal, that electric field is generated from this positive pole towards the negative pole, that negative ions are generated from the negative pole, and that static electricity is generated by movement of electrons or ions between substances when two different kinds of substances or similar kinds of substances are rubbed or separated. By using this phenomenon, the aforementioned object has been achieved.

Namely, the inventors have found out that, though the powder of an ore having spontaneous polarization does not generate polarization difference in a stationery state and does not generate negative ions, the powder generates negative ions though only slightly when static electricity is generated by friction, exfoliation, movement, or the like, and that if static electricity is forcibly given to a powder of such an ore having spontaneous polarization, a large amount of negative ions are generated. As a result of this, by allowing a powder of an ore having spontaneous polarization to be in contact with a substance charged with static electricity, the electric field balance of a crystal of an ore having spontaneous polarization is disturbed to generate polarization difference, thereby enabling generation of strong negative ions.

Therefore, as a result of various studies on substances to be brought in contact with a powder of an ore having spontaneous polarization, the inventors have found out that organofluororesin compounds are charged extremely strongly for a long time by small friction, exfoliation, or the like, and a desired result has been obtained by allowing the compound negatively charged to be in contact with a powder of an ore having spontaneous polarization.

When a substance is charged with static electricity, it is charged either positively or negatively. Examples of the organic compounds that are liable to be charged with positive static electricity include polyester resin, nylon resin, natural animal hair, polyvinyl chloride resin, and others. Examples of the organic compounds that are liable to be charged with negative static electricity include fluororesin, polyethylene resin, vinylon resin, acrylic resin, and others. The smaller the powder, particularly the particle size, is, the larger the surface area will be and the powder will be more liable to be charged with static electricity.

Allowing these substances to be charged easily with static electricity and to be charged freely with positive or negative electricity is made possible by a self-discharging method using electricity, a pulse direct current method, or stationery direct current method among voltage application methods, whereby an extremely high chargeability can be imparted. In particular, by the pulse direct current method, strong negative or positive high chargeability can be obtained more easily.

It has been found out that, though the state of normal substances being charged with static electricity decays in an extremely short time, some organofluororesin compounds have a peculiar nature of being electrically charged extremely strongly for a long time and, moreover, by the pulse direct current method using electricity, a strong high chargeability can be imparted easily and, in most of the cases, the compounds are negatively charged.

Therefore, in the present invention, by bringing a) powder of an ore having spontaneous polarization and b) a negatively charged organofluororesin compound into contact, the powder of the ore having spontaneous polarization generates a large amount of negative ions.

As powder of an ore having spontaneous polarization, it is preferable to use tourmaline powder or those containing the same. The organofluororesin compound may be used either after being electrically loaded to be negatively charged in advance, or may be electrically loaded to be negatively charged after being brought into contact or mixed with tourmaline powder. However, in view of the amount of negative ion discharge, it is efficient to use an organofluororesin compound negatively charged in advance as a mixture with powder of an ore having spontaneous polarization.

Generally, it is preferable that the ore having spontaneous polarization and the organofluororesin compound are both powder having a particle size of 1mm or less, and the mixing ratio thereof is 1/99 to 99/1. If the particle size of these powders exceeds 1 mm, the point of contact and the surface area decrease, so that the discharge of negative ions decreases and it is hard to obtain a desired result. It is especially preferable if the contact and mixing ratio of the ore powder having spontaneous polarization and the organofluororesin compound powder is 10 : 90 to 50 : 50.

Here, one may produce a resin molded article containing a mixture of ore powder having spontaneous polarization with an organofluororesin compound or a coating material containing ore powder having spontaneous polarization and an organofluororesin compound, and apply this material onto a base material such as cloth, paper, wood, plastics, or metal to prepare a negative ion generator. In this case, the organofluororesin compound may be negatively charged after the production of the resin molded article or after the application of the coating material.

Representative examples of the organofluororesin compounds to be used in the present invention include:
polytetrafluoroethylene ethylene copolymer resin,
polytetrafluoroethylene propylene copolymer resin,
polytetrafluoroethylene perfluoroalkylvinylether copolymer resin,
polytetrafluoroethylene hexafluoropropylene copolymer resin,
polytetrafluoroethylene hexafluoropropylene perfluoroalkylvinylether copolymer resin,
polytetrafluoroethylene resin,
polyvinyl fluoride resin,
polyvinylidene fluoride resin,
polychlorotrifluoroethylene resin, and
polychlorotrifluoroethylene ethylene copolymer resin.

A commercially available product of any one of these can be used.

When a person feels pain in the skin such as in a sprain, a bruise, muscle pain, or atopic dermatitis, the pain can be considerably relieved by applying to the corresponding site the treatment material containing the a) component and the b) component in contact as described above. Such a skin treatment material of the present invention may also be used in the following manner. A coating material containing the a) component and the b) component may be applied onto a sheet base material such as cloth or paper, and the treatment material may be used by applying it onto the affected skin part as a sheet-like treatment material. Alternatively, an aqueous liquid, an aqueous alcohol liquid, or the like containing the a) component and the b) component may be used as an agent to be applied to the affected part by the spray method or the like.

Here, representative examples of the organofluororesin compounds to be used in the above coating material include:
polytetrafluoroethylene ethylene copolymer resin,
polytetrafluoroethylene propylene copolymer resin,
polytetrafluoroethylene hexafluoropropylene copolymer resin,
polytetrafluoroethylene resin,
polyvinyl fluoride resin,
polyvinylidene fluoride resin,
polychlorotrifluoroethylene resin, and
polychlorotrifluoroethylene ethylene copolymer resin.

A commercially available product of any one of these can be used.

The definite reason why such a skin treatment material of the present invention is effective in alleviating and removing skin pain and in eliminating the atopic dermatitis is not known. However, it is known that, when a muscle is in tension, a large amount of electricity is generated, and when a wound is present, the corresponding site will have a positive potential, and accompanies a signal of pain. The negative ions or electrons generated from the skin treatment material of the present invention seem to function effectively by neutralizing the positive ions (signal of pain or the like).

Further, by putting powder of an ore having spontaneous polarization and powder of a negatively charged organofluororesin organic compound into a tubular rotary apparatus having a plurality of holes disposed therearound and rotating the apparatus, a product capable of generating negative ions in an extremely large amount can be made.

In this case, in order to prevent leakage of the powder mixture, it is preferable to attach a filter to the outer circumference (or the inner circumference) of the aforesaid rotary apparatus. It is preferable that the filter also contains powder of an ore having spontaneous polarization and a negatively charged organofluororesin organic compound, for example, by using a cloth to which a coating material containing the powder of the ore having spontaneous polarization and the negatively charged organofluororesin organic compound is applied, in the filter.

In particular, when the aforesaid rotary apparatus is made of aluminum, astonishing generation of negative ions can be achieved.

### BEST MODES FOR CARRYING OUT THE INVENTION

Next, the present invention will be described in more detail on the basis of the Examples and Comparative Examples. The appliance, apparatus, and others used in the Examples and the like are as follows.

### [static electricity load generating apparatus]

A pulse ionizer A-20J of the pulse direct current electricity method (manufactured by Suzuki Shokai in Kyoto) was used under the condition of an output direct current voltage of 5 kV, two tungsten electrode needles, an air outlet of 1/2 inch NTP, air pressure of 5 kgf/cm² for use, a distance of 10 cm between the electrode needle and the sample, and a radiation time of 5 seconds.

### [negative and positive ion generation performance evaluation]

Measurement was made by using an ionary measuring device SSI-1500 (manufactured by Suzuki Shokai in Kyoto) having a dynamic system air ion counter FIC-2000 (Manufactured by TOKYO FISA in Tokyo) mounted thereon as an ion measuring device. The measurement condition is as follows.

The measurement was made with a distance of 15 cm from the air suction inlet of a humidistat thermostat chamber air ion counter FIC-2000 at a temperature of 20°C and a humidity of 40% as a measurement atmosphere to the measurement sample, a vertical amplitude distance of the measurement sample of 15 cm, number of oscillation of 100 times/min (speed of 30 m/min) , and a measurement time of 1 minute. The measurement results show the maximum amount of negative ions and the maximum amount of positive ions. Average values of positive ions and negative ions of one minute were measured by the number of ions per 1 cc.

### Example 1

Amixture of 10 g of polytetrafluoroethylene resin (kaosilica TEE-100 manufactured by Tech Chem Institute: powder having an average particle size of 0.02 mm) - hereafter referred to as TFE powder - as an organofluororesin compound powder, which is charged with negative static electricity by a static electricity loading apparatus, and 90 g of black tourmaline powder (powder manufactured by Enex Co., Ltd. and having a particle size of 5 to 20 µm) was prepared and put into the inside of a double bag made of a smelted and bleached fine and high-density 100% cotton twill textile having a rectangular shape of 5 × 10 cm, which was then sealed before measurement of ion quantity.

### Examples 2 to 4

The ion quantity was measured in the same manner as in Example 1 except that the amount of the TFE powder and the amount of the black tourmaline powder were changed as follows.

### [Example 2] 50 g of TFE powder and 50 g of black tourmaline powder

### [Example 3] 75 g of TFE powder and 25 g of black tourmaline powder

### [Example 4] 90 g of TFE powder and 10 g of black tourmaline powder

### Example 5

The ion quantity was measured in the same manner as in Example 1 except that the TFE powder of Example 1 was changed to polytetrafluoroethylene hexafluoropropylene perfluoroalkylvinylether copolymer (kaosilica PPF-2300 manufactured by Tech Chem Institute: powder having an average particle size of 0.2 mm).

### Comparative Example 1

Only the black tourmaline powder used in Example 1 was put into the inside of a double bag made of a smelted and bleached fine and high-density 100% cotton twill textile having a rectangular shape of 5 × 10 cm, which was then sealed before measurement of ion quantity.

### Comparative Example 2

The ion quantity was measured in the same manner as in Comparative Example 1 by using black tourmaline powder imparted with negative static electricity.

### Comparative Example 3

Only the TFE powder used in Example 1 was put into the inside of a double bag made of a smelted and bleached fine and high-density 100% cotton twill textile having a rectangular shape of 5 × 10 cm, which was then sealed before measurement of ion quantity.

### Comparative Example 4

The ion quantity was measured in the same manner as in Comparative Example 3 by using TFE powder imparted with negative static electricity.

The results obtained in Examples 1 to 5 and Comparative Examples 1 to 4 are shown in Table 1.

**[Table 1]**

| Sample | Negative ion quantity | | Positive ion quantity, average value (2) | Difference in ion quantity: (1) - (2) |
|---|---|---|---|---|
| | Maximum value | Average value (1) | | |
| Example 1 | 1660 | 970 | 10 | 960 |
| Example 2 | 3730 | 2100 | 20 | 2080 |
| Example 3 | 11270 | 5970 | 20 | 5950 |
| Example 4 | 2510 | 1060 | 20 | 1040 |
| Example 5 | 5890 | 3320 | 120 | 3200 |
| Comparative Example 1 | 990 | 350 | 240 | 110 |
| Comparative Example 2 | 1040 | 610 | 350 | 260 |
| Comparative Example 3 | 920 | 580 | 130 | 450 |
| Comparative Example 4 | 710 | 390 | 240 | 150 |

### Example 6 - example in which a negative ion generator was allowed to adhere to a cotton textile

A stock solution obtained by mixing and uniformly stirring 5 parts by weight of polyurethane emulsion binder (Techcoat WH960 manufactured by Limited Company Furukawa Giken) , 3 parts by weight of a 10% aqueous solution of sodium alginate as a migration preventive agent, 1 part by weight of block isocyanate cross-linking agent (Super Fresh JB-7300 manufacturedbyLimitedCompany Furukawa Giken), and 0.2 part by weight of diphenyl ether anion surfactant penetrating agent (Pelex SSH manufactured by Kao Corp.) with 100 parts by weight of a 0.2% aqueous solution of polyacrylic resin viscous sedimentation preventing agent (Alcoprint PTF manufactured by Allied Co., Ltd. in Great Britain), was mixed with 4 parts by weight of black tourmaline powder (powder having an average particle size of 0.5 µm or less) and 11 parts by weight of a water dispersing element (kaosilica TEEC-45 manufactured by Tech Chem Institute) having a polytetrafluoroethylene ethylene copolymer resin concentration of 45% and containing 4% of a nonyl phenol surfactant. The resultant mixture was stirred for 5 minutes by a homodisper - 1000 rpm to prepare a treatment liquid.

A smelted and bleached pharmacopoeial gauze was immersed for 5 seconds into this treatment liquid, uniformly squeezed at a squeezing ratio of 80% with two mangles, dried at 100°C for 5 minutes, and thermally processed at 160°C for 2 minutes. Thereafter the gauze was washed with hot water of 50°C for 1 minute, squeezed, and dried at 100°C for 3 minutes.

The treatment gauze obtained in this manner was imparted with negative static electricity by a static electricity load generating apparatus to prepare a test piece.

The test piece was cut into strips of 10 cm × 2 cm, and these were stacked so that the stack would weigh 20 g ± 0.2 g. The long sides thereof at one end were bundled and bound, and those at the other end were not bundled. The strips were placed into a box of an ioner measurement device with the bundled sides up, and the ion quantity was measured with the aforementioned measurement device and measurement condition.

### Example 7

The ion quantity of a test piece was measured by carrying out a method similar to the one in Example 6 except that a water dispersing element (kaosilica TEEC-45L manufactured by Tech Chem Institute) having a polytetrafluoroethylene resin concentration of 45% was used instead of the polytetrafluoroethylene ethylene copolymer resin of Example 6.

### Comparative Example 5

A method similar to the one in Example 6 was carried out except that the process of imparting the treatment gauze with negative static electricity by a static electricity load generating apparatus was not carried out.

### Comparative Example 6

A method similar to the one in Example 6 was carried out except that a test piece was prepared by the method of Example 6 without using the polytetrafluoroethylene ethylene copolymer resin and that the process of imparting the treatment gauze with negative static electricity by a static electricity load generating apparatus was not carried out.

### Comparative Example 7

A method similar to the one in Example 6 was carried out except that a test piece was prepared by the method of Example 6 without mixing the black tourmaline powder and the polytetrafluoroethylene ethylene copolymer resin and that the process of imparting the treatment gauze with negative static electricity by a static electricity load generating apparatus was not carried out.

### Example 8

Into 100 parts by weight of long-oil phthalic acid solvent coating material (House Paint PRO white manufactured by Kampe Pavilio Co., Ltd.) were mixed 50 parts by weight of toluene, 50 parts by weight of acetone, 10 parts by weight of the powder of polytetrafluoroethylene perfluoroalkylvinylether copolymer resin (kaosilica TEFAB-1501 manufactured by Tech Chem Institute) having an average particle size of 20 µm and 3 parts by weight of pink tourmaline powder (powder manufactured by Enex and having an average particle size of 10 µm or less), and stirred to form a uniform mixture, which was then applied onto a wall paper having a commercially available paper quality, dried for 48 hours, and imparted with negative static electricity by a static electricity load generating apparatus in the same manner as in Example 6. A strip-like test piece was prepared and the ion quantity was measured.

### Comparative Example 8

A strip-like test piece was prepared without imparting negative static electricity to only the wall paper having a commercially available paper quality used in Example 8, and the ion quantity was measured.

### Comparative Example 9

A method similar to the one in Example 8 was carried out without mixing the powder of the polytetrafluoroethylene perfluoroalkylvinylether copolymer resin used in Example 8 and further without imparting negative static electricity.

The test results of Examples 6 to 8 and Comparative Examples 5 to 9 are shown in Table 2.

**[Table 2]**

| Test piece | Negative ion quantity | | Positive ion quantity, average value (2) | Difference in ion quantity: (1) - (2) |
|---|---|---|---|---|
| | Maximum value | Average value (1) | | |
| Example 6 | 4520 | 3350 | 40 | 3310 |
| Example 7 | 2420 | 1030 | 80 | 950 |
| Example 8 | 650 | 350 | 140 | 210 |
| Comparative Example 5 | 430 | 240 | 130 | 110 |
| Comparative Example 6 | 570 | 410 | 640 | -230 |
| Comparative Example 7 | 270 | 210 | 140 | 70 |
| Comparative Example 8 | 30 | 10 | 230 | -220 |
| Comparative Example 9 | 170 | 110 | 40 | 70 |

### Example 9

In order to supply negative ions to room air, a spray can was loaded with a mixture of 0.5 part by weight of tourmaline 0.5 µm (manufactured by Enex) , 1.2 parts by weight of a water dispersing element (kaosilica TEEC-45 manufactured by Tech Chem Institute) having a polytetrafluoroethylene ethylene copolymer resin concentration of 45%, 80 parts by weight of pure water, and 18.3 parts by weight of dimethyl ether, and the mixture was sprayed onto a box made of aluminum and connected to an ion measurement device from a distance of 1 meter for one second, and the ion quantity was measured.

### Example 10

A method similar to the one in Example 9 was carried out except that the distance between the spray can and the box was set to be 2 meters.

The test results of Examples 9 and 10 are shown in Table 3.

**[Table 3]**

| Sample | Negative ion quantity | | Positive ion quantity, average value (2) | Difference in ion quantity: (1) - (2) |
|---|---|---|---|---|
| | Maximum value | Average value (1) | | |
| Example 9 | 32430 | 8150 | 1652 | 6498 |
| Example 10 | 10260 | 2942 | 1105 | 1837 |

### Example 11

A mixture powder obtained by mixing 100 parts by weight of polytetrafluoroethylene resin (kaosilica TFE-1010 manufactured by Tech Chem Institute: average particle size of 0.02 mm) negatively charged by the above-mentioned method and 100 parts by weight of tourmaline having a particle size of 5 to 20 µm (manufactured by Enex Co., Ltd.), and the following filter were prepared.

The filter was prepared by applying a gray paste obtained by adding 7 parts by weight of polytetrafluoroethylene powder (kaosilica TFE-2200 manufactured by Tech Chem Institute) having an average particle size of 10 µm and negatively charged by the above-mentioned method and 4 parts by weight of black tourmaline powder having an average particle size of 3 µm (manufactured by Enex Co., Ltd.) to 100 parts by weight of acrylic resin binder containingmineral terpene fiber pigment printing paste (Unibinder MCO manufactured by Uni Kasei Co., Ltd.: white paste having a viscosity of 21000 cps) and stirring and mixing for 10 minutes with a homodisper of 2000 rpm and having a viscosity of 48000 cps, onto the whole front surface of a cotton cloth with a silk screen, and dried for 6 hours. Thereafter, a similar paste was applied again, and dried for 12 hours. The back surface was also treated with the same process as the front surface.

This filter was fixed onto the outer circumference of a box body made of aluminum (a tube having a length of 20 cm and a diameter of 6.5 cm, having 72 holes of 2 cm diameter therearound, and being rotatable with its central axis serving as a rotation axis) so that the contents would not leak out. The above-described mixture powder was put into the box body, which was then tightly closed with a lid, and rotated at 3 revolutions per second around the aforementioned axis, and the ion quantity was measured at a distance of 30 cm from the ionary measurement device.

### Example 12

A method similar to the one in Example 12 was carried out except that a wooden box body was used instead of the box body made of aluminum of Example 11.

The test results of Examples 11 to 12 are shown in Table 4.

**[Table 4]**

| Sample | Negative ion quantity | | Positive ion quantity, average value (2) | Difference in ion quantity: (1) - (2) |
|---|---|---|---|---|
| | Maximum value | Average value (1) | | |
| Example 11 | 50010 | 31931 | 9674 | 22257 |
| Example 12 | 2060 | 1115 | 16 | 1099 |

### Example 13

Into a 2-liter glass vessel, 40 g of polytetrafluoroethylene resin powder (kaosilica TFE-100 manufactured by Tech Chem Institute) having a particle size of 1 to 0.02 mm as organofluororesin compound powder, 40 g of black tourmaline powder having a particle size of 0.5 to 10 µm, 300 g of a water-dispersing resin made of acrylic acid copolymer containing butyl acrylate as a major component and having a viscosity of 50000 CPS/20°C and a concentration of 45% as a binder, and 620 g of water were put and mixed and stirred for 5 minutes with a hand mixer of 800 rpm to obtain a uniform gray viscous mixture.

This viscous mixture was uniformly applied onto one side of a smelted and bleached 100% cotton two-side napped flannelette textile at a ratio of 120 g/m² directly by the print method and, after natural drying for 24 hours, subjected to iron pressing at 150°C for 30 seconds. Finally, negative charging was carried out with a pulse ionizer A-20J of the pulse direct current electricity method (manufacturedby Suzuki Shokai in Kyoto) under the condition of an output direct current voltage of 5 kV, two tungsten electrode needles, an air outlet of 1/2 inch NTP, air pressure of 5 kgf/cm² for use, a distance of 10 cm between the electrode needle and the sample, and a radiation time of 5 seconds to obtain a sheet-like treatment material of Example 1.

The charged voltage of this material was measured with a static control TI-300 made by Richmond at a temperature of 22°C, a humidity of 52% and a distance of 10 cm, showing not less than minus 10000 V. Further, this was measured with an air ion concentration measuring device FIC-2000 made by FISA at a temperature of 22°C, a humidity of 52%, a size of the measurement sample being 30 cm square, a vertical amplitude of 20 cm, and a rate of 240 times/minute, showing a negative ion quantity of 3240/cc and a positive ion quantity of 186/cc. Thus, the generation of a large amount of negative ions from the sheet-like treatment material was confirmed.

The obtained sheet-like treatment material was put into an aluminum sheet-like bag immediately after preparation, and stored until just before use.

### Example 14

A liquid treatment material A was prepared with a mixture of 0.5 part by weight of 0.5 µm tourmaline, 1.2 parts by weight of a water dispersing element (kaosilica TEEC-45 manufactured by Tech Chem Institute) having a polytetrafluoroethylene ethylene copolymer resin concentration of 45%, 80 parts by weight of pure water, and 18.3 parts by weight of dimethyl ether. Onto a gauze, the liquid treatment material having the same weight as the gauze was sprayed and allowed to adhere and, after natural drying for 24 hours, a home iron was applied at about 150°C for 10 seconds. Finally, negative charging was carried out with a pulse ionizer A-20J of the pulse direct current electricity method (manufactured by Suzuki Shokai in Kyoto) under the condition of an output direct current voltage of 5 kV, two tungsten electrode needles, an air outlet of 1/2 inch NTP, air pressure of 5 kgf/cm² for use, a distance of 10 cmbetween the electrode needle and the sample, and a radiation time of 5 seconds to obtain a sheet-like treatment material B of Example 2.

### [examples of use of the treatment materials obtained in Examples 1 to 2]

### Use Example 1

The viscous mixture applied surface of the treatment material obtained in Example 1 was applied to an affected area of the site swollen in blue by internal bleeding of a patient that is unsteady in walking by having stepped off from the steps to suffer from ankle sprain. Almost all the pain disappeared in one night, and the patient could walk freely. Also, the swell disappeared in two or three days.

### Use Example 2

The viscous mixture applied surface of the treatment material obtained in Example 1 was applied to the scruff of the neck of a 12-year old girl with her head hit hard and suffering from whiplash symptoms after school, stiff scruff of the neck, and being incapable of freely rotating her head, and also having a severe headache. The pain disappeared in about 30 minutes and, in the next morning, the pain and the stiffness of the scruff of the neck disappeared, thereby regaining her usual normal life.

### Use Example 3

The treatment material obtained in Example 1 was applied to the waist of a woman in seventies suffering from a chronic lumbago for many years. Although the pain did not disappear completely, the lady was considerably eased. In particular, though it had been painful to walk due to the pain, the pain was alleviated, and it was no longer painful to walk.

### Use Example 4

A sixty-three year old woman suffering from insomnia for many years and taking two tablets of sleep aiding melatorin every day used the treatment material obtained in Example 1 as a pillow cover. She became able to sleep profoundly without the sleep aid.

### Use Example 5

A man in fifties who is wakeful and unable to sleep profoundly because of waking up at night used the treatment material obtained in Example 1 as a pillow cover. He became less wakeful and became able to sleep profoundly.

### Use Example 6

The treatment material B obtained in Example 2 was attached for use to pyjamas of a nine year old boy having a dry skin, suffering from itchy skin, and having his skin stained with blood because of scratching at all times. The dry skin became smooth in two days; the itchiness disappeared; and he became able to sleep profoundly. Thereafter, he became able to sleep profoundly at all times.

### Use Example 7

The liquid treatment material A of Example 2 was sprayed and applied to cotton pyjamas of seven year old and eleven year old children having atopy in the back. Negative charging was carried out with a pulse ionizer A-20J of the pulse direct current electricity method (manufactured by Suzuki Shokai in Kyoto) under the condition of an output direct current voltage of 5 kV, two tungsten electrode needles, an air outlet of 1/2 inch NTP, air pressure of 5 kgf/cm² for use, a distance of 10 cm between the electrode needle and the sample, and a radiation time of 5 seconds. The charged voltage of this was measured with a static control TI-300 made by Richmond at a temperature of 22°C, a humidity of 52% and a distance of 10 cm, showing not less than minus 10000 V. Further, this was measured with an air ion concentration measuring device FIC-2000 made of FISA at a temperature of 22°C, a humidity of 52%, a size of the measurement sample being 30 cm square, a vertical amplitude of 20 cm, and a rate of 240 times/minute, showing a negative ion quantity of 1240/cc and a positive ion quantity of 213/cc. Thus, the generation of a large amount of negative ions from the pyjamas was confirmed.

The children were allowed to use these pyjamas. After two weeks, the itchiness disappeared, and the skin became clean.

### INDUSTRIAL APPLICABILITY

The product of the present invention gives substantially no fear of discharging radiation, and discharges negative ions stably and efficiently for a long period of time, so that it is extremely easy to be handled, and a variety of commercial development of the product can be made. Further, the skin treatment material using this negative ion generator is extremely useful for eliminating the pain of skin (sprain, muscle pain, bruise, chronic pain, atopic dermatitis, and others) by being applied to the skin as a sheet-like treatment material or by being sprayed and applied as a liquid treatment material.

## Claims

1. A negative ion generator **characterized by** containing a) powder of an ore having spontaneous polarization and b) an organofluororesin organic compound in a contact state, where b) component is negatively charged.

2. The negative ion generator according to claim 1, **characterized in that** a) component contains tourmaline powder.

3. The negative ion generator according to claim 1 or 2, **characterized in that** b) component contains fluororesin organic compound powder negatively charged by electrical charging.

4. The negative ion generator according to any one of claims 1 to 3, **characterized in that** a) component and b) component are both powder having a particle size of 1 mm or less, and that a mixing ratio of a) component and b) component is 1/99 to 99/1.

5. The negative ion generator according to any one of claims 1 to 4, **characterized by** being composed of a resin molded article containing a) component and b) component.

6. The negative ion generator according to any one of claims 1 to 4, **characterized in that** a coating material containing a) component and b) component is applied onto a base material.

7. The negative ion generator according to any one of claims 1 to 4, **characterized in that** a rotary container made of aluminum and having a plurality of holes disposed therearound is filled with a mixture of a) component and b) component, and is used by rotating.

8. The negative ion generator according to any one of claims 1 to 7, **characterized in that** said fluororesin organic compound is selected from the group consisting of:
polytetrafluoroethylene ethylene copolymer resin,
polytetrafluoroethylene propylene copolymer resin,
polytetrafluoroethylene perfluoroalkylvinylether copolymer resin,
polytetrafluoroethylene hexafluoropropylene copolymer resin,
polytetrafluoroethylene hexafluoropropylene perfluoroalkylvinylether copolymer resin,
polytetrafluoroethylene resin,
polyvinyl fluoride resin,
polyvinylidene fluoride resin,
polychlorotrifluoroethylene resin, and
polychlorotrifluoroethylene ethylene copolymer resin.

9. A skin treatment material **characterized by** containing a) powder of an ore having spontaneous polarization and b) an organofluororesin organic compound in a contact state.

10. The skin treatment material according to claim 9, **characterized in that** a) component contains tourmaline powder.

11. The skin treatment material according to claim 9 or 10, **characterized in that** b) component containsfluororesin organic compound powder negatively charged by electrical charging.

12. The skin treatment material according to any one of claims 9 to 12, **characterized in that** a) component and b) component are both powder having a particle size of 1 mm or less, and that a mixing ratio of a) component and b) component is 1/99 to 99/1.

13. The skin treatment material according to any one of claims 9 to 12, **characterized in that** a coating material containing a) component andb) component is applied onto a sheet basematerial.

14. The skin treatment material according to any one of claims 9 to 12, **characterized by** being a coating agent composed of aqueous liquid containing a) component and b) component.

15. The skin treatment material according to any one of claims 9 to 14, **characterized in that** said fluororesin organic compound is selected from the group consisting of:
polytetrafluoroethylene ethylene copolymer resin,
polytetrafluoroethylene propylene copolymer resin,
polytetrafluoroethylene hexafluoropropylene copolymer resin,
polytetrafluoroethylene resin,
polyvinyl fluoride resin,
polyvinylidene fluoride resin,
polychlorotrifluoroethylene resin, and
polychlorotrifluoroethylene ethylene copolymer resin.
